(19) Europäisches Patentamt
European Patent Office
Office européen des brevets

(11) EP 0 521 353 B1

(12) **EUROPÄISCHE PATENTSCHRIFT**

(45) Veröffentlichungstag und Bekanntmachung des Hinweises auf die Patenterteilung:
**17.04.1996 Patentblatt 1996/16**

(51) Int. Cl.$^6$: **A61K 31/685**

(21) Anmeldenummer: **92110347.9**

(22) Anmeldetag: **19.06.1992**

(54) **Arzneimittel mit antineoplastischer Wirkung enthaltend als Wirkstoff Octadecyl-[2-(N-methylpiperidino)-ethyl]-phosphat und Verfahren zu dessen Herstellung**

Antineoplastic medicament containing octadecyl-[2-(N-methylpiperidino)-ethyl] phosphate and synthesis thereof

Medicament antinéoplastique contenant du octadécyl-[2-(N-méthyl pipéridino)-éthyl] phosphate, et synthèse du même

(84) Benannte Vertragsstaaten:
**AT BE CH DE DK ES FR GB GR IT LI LU MC NL PT SE**

(30) Priorität: **04.07.1991 DE 4122140**
**04.09.1991 DE 4129364**

(43) Veröffentlichungstag der Anmeldung:
**07.01.1993 Patentblatt 1993/01**

(73) Patentinhaber: **ASTA Medica Aktiengesellschaft
D-01277 Dresden (DE)**

(72) Erfinder:
- **Schumacher, Wolfgang, Dr.
W-6070 Langen (DE)**
- **Engel, Jürgen, Prof.
W-8755 Alzenau (DE)**
- **Nössner, Gerhard, Dr.
W-6050 Offenbach (DE)**
- **Kutscher, Bernhard, Dr.
W-6457 Maintal 1 (DE)**
- **Stekar, Jurij, Dr.
W-4800 Bielefeld 13 (DE)**
- **Hilgard, Peter, Dr.
W-4800 Bielefeld 1 (DE)**

(56) Entgegenhaltungen:
**EP-A- 0 108 565**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 521 353 B1

Printed by Rank Xerox (UK) Business Services
2.11.2/3.4

**Beschreibung**

Die Europa-Patentanmeldung 108 565 betrifft Verbindungen der allgemeinen Formel

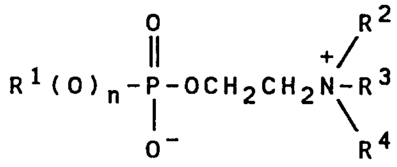

und deren pharmazeutisch verwertbare Salze, worin $R_1$ ein aliphatischer Kohlenwasserstoffrest mit 8 - 30 C-Atomen ist, die Reste $R_2$, $R_3$ und $R_4$ gleich oder verschieden sind und Wasserstoff oder niedere Alkylreste sind, oder worin die Gruppe $NR_2R_3R_4$ eine cyclische Ammoniumgruppe bedeutet und n die Werte 0 oder 1 hat. Für diese Verbindungen wird eine Anti-Tumor-Wirkung sowie eine pilzhemmende Wirkung angegeben.

Die Erfindung betrifft das Octadecyl-[2-(N-methylpiperidino)-ethyl]-phosphat, Verfahren zu dessen Herstellung sowie Arzneimittel, die als Wirkstoff diese Verbindung enthalten. Überraschenderweise besitzt die erfindungsgemäße Verbindung eine sehr viel bessere beziehungsweise vorteilhaftere Antitumorwirkung als die in der Europa-Anmeldung 108 565 beschriebenen beziehungsweise erwähnten Verbindungen.

Außerdem bewirken die erfindungsgemäßen Verbindungen eine Hemmung der Blutblättchenaggregation; weiterhin besitzen sie eine Phospholipase-$A_2$-hemmende sowie eine Lipoxygenase-hemmende Wirkung.

Die Erfindung betrifft auch ein neues, verbessertes Herstellungs- und Aufarbeitungsverfahren für das Octadecyl-[2-(N-methylpiperidino)-ethyl] -phosphat (Verfahren a).

Überraschenderweise wurde gefunden, daß durch das erfindungsgemäße Verfahren eine höhere Gesamtausbeute und ein reineres Produkt erzielt wird, obwohl ein Reinigungsschritt weniger als in den vorbekannten Verfahren für Alkylphosphocholine angewandt wird. Weiterhin wird hier weniger Lösungsmittel verwendet. Das neue erfindungsgemäße Verfahren vermeidet auch die Verwendung von alkylierenden Reagentien, wie z. B. Dimethylsulfat, die durch die Verwendung von Kaliumkarbonat als Hilfsbase zu einem hohen Kaliumgehalt des Produkts führen. Der Kaliumgehalt muß bei Substanzen, die als pharmazeutische Wirkstoffe eingesetzt werden, möglichst gering sein.

Ebenfalls ist der zeitaufwendige Chromatographieschritt für die Reinigung nicht mehr erforderlich.

Die gemäß dem neuen Verfahren erzielte Produktreinheit ist höher als bei den bekannten Verfahren zur Herstellung von Alkylphosphocholinen, außerdem liefert es höhere Ausbeuten (insbesondere bei der Herstellung in größerem Maßstab).

Die erste Stufe der des Verfahrens a) besteht in der Reaktion von Phosphoroxychlorid mit N-Octadecylalkohol in halogenierten Kohlenwasserstoffen, gesättigten cyclischen Ethern, acyclischen Ethern, gesättigten Kohlenwasserstoffen mit 5 bis 10 C-Atomen, flüssigen aromatischen Kohlenwasserstoffen, die auch durch Halogen (insbesondere Chlor) substituiert sein können oder in Gemischen aus den vorstehend erwähnten Lösungsmitteln oder ohne Lösungsmittel, gegebenenfalls in Gegenwart einer hierfür üblichen basischen Substanz.

Als halogenierte Kohlenwasserstoffe kommen beispielsweise Kohlenwasserstoffe mit 1 bis 6 C-Atomen in Frage, wobei ein oder mehrere oder alle Wasserstoffatome durch Chloratome ersetzt sind. Beispielsweise können Methylenchlorid, Chloroform, Ethylenchlorid, Chlorbenzol, Dichlorbenzol verwendet werden. Falls es sich um halogensubstiuierte aromatische Kohlenwasserstoffe handelt, sind diese vorzugsweise mit einem oder zwei Halogenatomen substituiert.

Als gesättigte cyclische Ether können beispielsweise Ether mit einer Ringgröße von 5-6. die aus Kohlenstoffatomen und einem oder 2 Sauerstoffatomen bestehen, verwendet werden.
Beispiele hierfür sind Tetrahydrofuran, Dioxan.

Die acyclischen Ether bestehen aus 2 bis 8 Kohlenstoffatomen und sind flüssig. Beispielsweise kommen in Frage: Diethylether, Diisobutylether, Methyl-tert.-butylether, Disopropylether.

Als gesättigte Kohlenwasserstoffe kommen unverzweigte und verzweigte Kohlenwasserstoffe, die aus 5 bis 10 Kohlenstoffatomen bestehen und flüssig sind, in Frage. Beispielsweise kommen Pentan, Hexan, Heptan, Cyclohexan, in Frage.

Als aromatische Kohlenwasserstoffe kommen beispielsweise Benzol und alkylsubstituierte Benzole, wobei die Alkylsubstituenten aus 1 bis 5 Kohlenstoffatomen bestehen, in Frage.

Als basische Substanzen sowohl für die Reaktion des Phosphoroxychlorids mit dem n-Octadecylalkohol als auch für die anschließende Umsetzung mit dem N-Methyl-piperidino-ethanol-Salz kommen Amine in Frage, beispielsweise aliphatische

Amine der Formel $NR_1R_2R_3$, wobei $R_1$, $R_2$ und $R_3$ gleich oder verschieden sind und Wasserstoff oder $C_1$-$C_6$-Alkyl bedeuten oder auch aromatische Amine wie Pyridin, Picolin, Chinolin.

Bei der Umsetzung mit dem N-Methyl-piperidino-ethanol-Salz kann die hier erforderliche basische Substanz gleichzeitig

oder auch vor dem N-Methyl-piperidino-ethanol-Salz zugesetzt werden. Für diese Reaktion ist in jedem Fall ein Lösungsmittel erforderlich; das heißt falls der erste Reaktionsschritt ohne ein besonderes Lösungsmittel erfolgt, muß ein solches jetzt zugegeben werden. Das Molverhältnis von Phosphoroxichlorid zu dem Octadecylalkohol liegt zum Beispiel zwischen 1,5:1 bis 1:1.

Das N-Methyl-piperidino-ethanol-Salz wird beispielsweise in Bezug auf den n-Octadecylalkohol im Überschuß eingesetzt (etwa 1,1 - 1,5 molarer Überschuß).

Falls die Reaktion des Phosphoroxichlorids mit dem n-Octadecylalkohol in Gegenwart einer basischen Substanz erfolgt, ist die Menge der basischen Substanz beispielsweise 1 bis 3 Mol bezogen auf 1 Mol $POCl_3$. Für die anschließende Umsetzung mit dem Piperidinsalz* ist die verwendete Menge an basischer Substanz beispielsweise 1 bis 5 Mol bezogen auf 1 Mol Alkanol.

Die Reaktionstemperatur der Umsetzung von Phosphoroxychlorid mit n-Octadecylalkohol liegt zwischen -30°C und +30°C, vorzugsweise -15°C und +5°C insbesondere -10°C und -5°C.

Die Reaktionszeit dieser Umsetzung beträgt beispielsweise 0,5-5 Stunden, vorzugsweise 1-3 Stunden, insbesondere 1,5-2 Stunden. Falls die Reaktion in Gegenwart einer basischen Substanz erfolgt, verläuft sie im allgemeinen schnell (etwa 30 Minuten).

Danach wird das Piperidinsalz portionsweise oder vollständig zugegeben.
Als Salze des N-Methyl-piperidin-ethanols kommen Salze mit Mineralsäuren, (wie zum Beispiel Schwefelsäure, Salzsäure) in Frage, ferner Salze mit organischen Säuren, wie z. B. Essigsäure, para-Toluolsulfonsäure und ähnliche.
Dieser Reaktionsschritt erfolgt in einem inerten Lösungsmittel. Als Lösungsmittel kommen hier die gleichen in Frage, die für die Umsetzung des Phosphoroxychlorids mit dem n-Octadecylalkohol verwendet werden, falls diese Umsetzung in einem Lösungsmittel erfolgt.

Anschließend wird die basische Substanz in einem der angegebenen Lösungsmittel gelöst, oder ohne Lösungsmittel zugetropft.
Vorzugsweise werden als Lösungsmittel für die basische Substanz hier verwendet: Halogenierte Kohlenwasserstoffe, gesättigte cyclische Ether, acyclische Ether, gesättigte Kohlenwasserstoffe mit 5 bis 10 Kohlenstoffatomen, flüssige aromatische Kohlenwasserstoffe oder Gemische aus vorstehend erwähnten Lösungsmitteln.
Es handelt sich hier um die gleichen Lösungsmittel, wie sie für die Umsetzung des Phosphoroxidchlorids mit dem n-Octadecylalkohol verwendet werden können.

Durch die Zugabe der basischen Substanz steigt die Temperatur. Man sorgt dafür, daß die Temperatur in einem Bereich zwischen 0°C bis 40°C, vorzugsweise 10°C bis 30°C, insbesondere auf 15°C bis 20°C gehalten wird.

Die Reaktionsmischung wird dann noch bei 5°C bis 30°C, vorzugsweise 15°C und 25°C gerührt, (beispielsweise 1 Stunde bis 40 Stunden, vorzugsweise 3 Stunden bis 15 Stunden.)

Die Hydrolyse des Reaktionsansatzes erfolgt durch Zugabe von Wasser, wobei eine Temperatur zwichen 10°C und 30°C, vorzugsweise 15°C und 30°C, insbesondere zwischen 15°C und 20°C eingehalten werden soll.

Die zuvor erwähnten Hydrolyseflüssigkeiten können auch basische Substanzen enthalten. Als solche basischen Substanzen kommen Carbonate und Hydrogencarbonate der Alkali- und Erdalkalimetalle in Frage.

Zur Vervollständigung der Hydrolyse wird anschließend noch 0,5 Stunden bis 4 Stunden, vorzugsweise 1 bis 3 Stunden, insbesondere 1,5 bis 2,5 Stunden bei 10°C bis 30°C, vorzugsweise bei 15°C bis 25°C, insbesondere bei 18°C bis 22°C gerührt.

Die Reaktionslösung wird dann mit einem Gemisch aus Wasser und Alkoholen (vorzugsweise aliphatische gesättigte Alkohole mit 1 bis 4 Kohlenstoffatomen), das gegebenenfalls noch eine basische Substanz erhalten kann, gewaschen. Das Mischungsverhältnis Wasser:Alkohol kann beispielsweise zwischen 5 und 0,5 liegen, vorzugsweise 1-3 (V/V).

Als basische Substanzen für die Waschflüssigkeit kommen zum Beispiel Carbonate und Hydrogencarbonate der Alkali- und Erdalkalimetalle sowie Ammoniak (zum Beispiel wäßriges Ammoniak) in Frage. Besonders bevorzugt ist eine 3%ige Natriumkarbonatlösung in Wasser.

Gegebenenfalls kann anschließend eine Waschung der Reaktionslösung mit einer sauren Lösung vorgenommen werden.
Vorteilhaft ist die saure Waschung zur Entfernung noch nicht umgesetzter basischer Anteile der Reaktionslösung, insbesondere bei der Verwendung von Methylenchlorid als Lösungsmittel.

Die Waschlösung besteht aus einem Gemisch aus Wasser und Alkoholen. Vorzugsweise kommen Mischungen aus aliphatischen gesättigten Alkoholen mit 1 bis 4 Kohlenstoffatomen in Frage, wobei gegebenenfalls noch eine saure Substanz anwesend sein kann. Das Mischungsverhältnis Wasser:Alkohol kann beispielsweise zwischen 5 und 0,5 liegen, vorzugsweise 1-3 (V/V).

Als saure Substanz für die Waschflüssigkeit kommen zum Beispiel Mineralsäuren und organische Säuren in Frage, zum Beispiel Salzsäure, Schwefelsäure oder Weinsäure, und Zitronensäure. Besonders bevorzugt ist eine 10%ige Lösung von Salzsäure in Wasser.

* Piperidinsalz bedeutet stets N-Methyl-piperidino-ethanol-Salz

Anschließend wird noch einmal mit einem Gemisch aus Wasser und Alkoholen gewaschen. Vorzugsweise kommen Mischungen aus aliphatischen gesättigten Alkoholen mit 1 bis 4 Kohlenstoffatomen in Frage, wobei gegebenenfalls noch eine basische Substanz anwesend sein kann.
Das Mischungsverhältnis Wasser: Alkohol kann beispielsweise zwischen 5 und 0,5 liegen, vorzugsweise 1-3.

Die gewaschenen Phasen werden dann vereinigt und in üblicher Weise getrocknet, und dann das Lösungsmittel (vorzugsweise unter vermindertem Druck, zum Beispiel 5 bis 100 mbar) gegebenenfalls nach Zugabe von 150 -1000 ml, vorzugsweise 300 - 700 ml, insbesondere 450 -550 ml eines aliphatischen Alkohols entfernt (bezogen auf 1 Mol Gewichtsteil trocknes Produkt). Als Alkohole kommen vorzugsweise gesättigte aliphatische Alkohole mit einer Kettenlänge von 1 und 5 Kohlenstoffatomen in Frage. Besonders bevorzugt als Alkohol ist hierbei n-Butanol, Isopropanol. Diese Alkoholbehandlung hat den Zweck der vollständigen Entfernung von Restwasser.

Das so erhaltene Produkt kann in der üblichen Weise (z.B. durch Chromatographie, Umkristallisieren) gereinigt werden.

Besonders vorteilhaft ist jedoch das folgende, ebenfalls erfinderische Reinigungsverfahren:
Der wie oben beschriebene feste Rückstand wird beispielsweise in gesättigten aliphatischen Ketonen (3-6 Kohlenstoffatome), beispielsweise Aceton, Butanon, Methyl-tert.-butylketon aufgeschlämmt, 1 bis 4 Stunden, vorzugsweise 2 Stunden gerührt, abgesaugt und bei 20°C bis 50°C im Vakuum bei 5 Torr bis 100 Torr getrocknet.

Das so vorgereinigte Produkt wird in wasserfreien Alkoholen ($C_1$ bis $C_4$) beziehungsweise solchen Alkoholen, die höchstens bis zu 5 Gewichts-% Wasser enthalten bei 20°C bis 60°C, vorzugsweise 40°C aufgenommen und vom Unlöslichen abfiltriert. Als Alkohole werden beispielsweise Methanol, Ethanol, Isopropanol, Butanol, Isobutanol verwendet. Das erhaltene Filtrat wird dann mit einem Mischbett-Ionenaustauscher, zum Beispiel Amberlite® MB3 beispielsweise 1 bis 5 Stunden, vorzugsweise 2 Stunden bei 10°C bis 50°C, vorzugsweise 20°C gerührt.
Anstelle eines Mischbett-Ionenaustauschers kann die Reinigung auch nacheinander mit einem sauren Ionenaustauscher und einem basischen Ionenaustauscher erfolgen.

Als Ionenaustauscher können alle unlösliche Feststoffe, die ionenaustauschende Gruppen enthalten, verwendet werden.

Saure Ionenaustauscher sind solche, die z.B. saure Gruppen, wie Sulfonsäuregruppen, Carboxygruppen enthalten. Beispiele sind Ionenaustauscher mit Sulfonsäuregruppen an einer Polystyrolmatrix, wie zum Beispiel
Amberlite® IR 120, Dowex® HCR,
Duolite® C 20 oder Lewatit® S 100.
Schwach saure Ionenaustauscher sind zum Beispiel solche, auf Basis einer Polyacrylsäure-Matrix, wie z. B. Amberlite® IRC 76, Duolite® C 433
oder Relite® CC.

Als basische Ionenaustauscher kommen beispielsweise solche in Frage, die an einer Polymer-Matrix (z.B. Polystyrole-Matrix) primäre, sekundäre, tertiäre oder quartäre Aminogruppen tragen, wie zum Beispiel
Duolite® A 101, Duolite® A 102,
Duolite®15 A 348, Duolite A® 365,
Duolite® A 375, Amberlite® IRA 67,
Duolite® A 375, Amberlite® IRA 458 und
Duolite® A 132.

Mischbettionenaustauscher sind Gemische aus sauren und alkalischen Ionenaustauscherharzen, wie zum Beispiel
Amberlite® MB1, Amberlite® MB2,
Amberlite® MB3 und Amberlite® MB6.

Weitere verwendbare Ionenaustauscher sind in Ullmann's Encyclopedia of Industrial Chemistry, 5. Auflage (1989), Band A14, S. 450 aufgeführt.

Nach dem Absaugen vom Ionenaustauscherharz wird unter vermindertem Druck (beispielsweise 20 Torr bis 200 Torr) bei 40°C bis 70°C eingedampft und anschließend aus halogenierten Kohlenwasserstoffen, gesättigten aliphatischen Ketonen oder aus Alkohol/Keton-Gemischen umkristallisiert.

Als halogenierte Kohlenwasserstoffe für die Umkristallisation kommen beispielsweise Kohlenwasserstoffe aus 1 bis 6 C-Atomen in Frage, wobei ein oder mehrere oder alle Wasserstoffatome durch Chloratome ersetzt sind. Beispielsweise können Methylenchlorid, Chloroform, Ethylenchlorid, Chlorbenzol verwendet werden.

Als Alkohole kommen gesättigte aliphatische Alkohole mit 1 bis 6 Kohlenstoffatomen und 1 bis 3 Hydroxylgruppen in Betracht. Als Ketone kommen gesättigte, aliphatische Ketone mit 3 bis 6 Kohlenstoffatomen in Betracht.

Das Mischungsverhältnis Alkohol:Keton beträgt 1 zu 1-5 (Volumen/Volumen).
Besonders bevorzugt ist ein Ethanol/Aceton-Gemisch im Verhältnis 1 : 1 (V/V).

Die erhaltenen Kristalle des Octadecyl-[2-(N-methyl-piperidino)- ethanol-phosphats werden abgesaugt und, falls erforderlich, beispielsweise mit gesättigten Kohlenwasserstoffen mit 1 bis 6 Kohlenstoffatomen gewaschen. (Temperatur der Waschflüssigkeit z.B. 15 bis 30°C).

Weiterhin kann man die erfindungsgemäße Verbindung dadurch erhalten, daß man in an sich bekannter Weise

b) eine Verbindung der Formel

$$C_{18}H_{37}\text{-O-PO(OH)}_2 \qquad I$$

oder ein reaktionsfähiges Derivat dieser Verbindung mit einer Verbindung der Formel

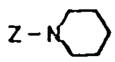

II

gegebenenfalls in Gegenwart basischer Substanzen umsetzt, worin Z Wasserstoff, Methyl oder die Gruppe $-CH_2-CH_2-OH$ darstellt und die Piperidinverbindung auch in Form des N-Methyl-piperidinium Derivats vorliegen kann, wobei in diesem Falle die positive Ladung durch das Anion einer anorganischen oder organischen Säure neutralisiert wird und gegebenenfalls methyliert, oder

c) eine Verbindung der Formel

$$C_{18}H_{37}\text{-O-PO(OH)-O-}CH_2\text{-}CH_2\text{-Y} \qquad III$$

worin Y Chlor, Brom oder Jod ist mit Piperidin oder N-Methyl-piperidin umsetzt und gegebenenfalls methyliert oder

d) eine Verbindung der allgemeinen Formel

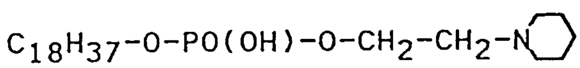

IV

methyliert, beziehungsweise eine gemäß b) oder c) erhaltene Verbindung methyliert.

Zu dem Verfahren b):

Das Verfahren b) kann ohne Lösungsmittel (in diesem Fall Überschuß an Verbindung II) oder in einem inerten Lösungsmittel bei Temperaturen zwischen 0 und 180 °C, vorzugsweise 18- 120°C durchgeführt werden. Als Lösungsmittel eigenen sich beispielsweise: niedere aliphatische Alkohole (Methanol), aliphatische geradkettige Ether (Diethylether), gesättigte cyclische Ether (Dioxan, Tetrahydrofuran), aromatische Kohlenwasserstoffe (Toluol, Benzol, Pyridin), chlorierte aliphatische niedermolekulare Kohlenwasserstoffe ($CHCl_3$, $CCl_4$), niedere Alkylamide oder Dialkylamide von niedermolekularen aliphatischen Carbonsäuren (Dimethylformamid, Dimethylacetamid) oder auch Dimethylsulfoxid, Acetonitril. Die Aufarbeitung erfolgt in bekannter Weise durch Hydrolyse mittels Alkoholen/Wasser oder Wasser allein. Reinigung des Reaktionsproduktes erfolgt beispielsweise durch Umkristallisieren oder Säulenchromatographie.

Die Umsetzung gemäß dem Verfahren b) kann auch in Gegenwart zusätzlicher basischer Substanzen wie zum Beispiel tertiären Aminen (N-Methyl-piperidin, N-Methyl-morpholin, N-Methyl-pyrrolidin, Chinolin, Pyridin) erfolgen.

Falls die Ausgangsverbindung die freie Säure $C_{18}H_{37}\text{-O-PO(OH)}_2$ ist, wird diese durch bekannte Kondensationsmittel wie z. B. 2,4,6-Trimethylbenzolsulfonylchlorid, 8-Chinolinsulfonylchlorid, 2,4,6-Isopropylbenolsulfonylimidazolid, 2,4,6-Trimethylbenzolsulfonyltetrazolid oder Dicyclohexylcarbodiimid aktiviert und dann umgesetzt.

Falls die Ausgangsverbindung $C_{18}H_{37}\text{-O-PO(OH)}_2$ in Form eines aktivierten Derivates vorliegt, handelt es sich beispielsweise um solche Verbindungen, wo beide OH-Gruppen durch Halogen ersetzt sind (Cl, Br, J),

Als aktiviertes Derivat kann z. B. auch eine Verbindung der Formel

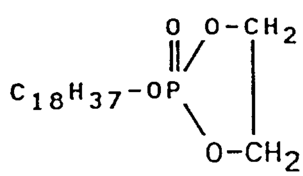

eingesetzt werden: in diesem Falle erfolgt die Reaktion vorzugsweise in einem Autoklaven oder einer Rührapparatur bei einer Temperatur zwischen -20 bis 130°C.

Falls die Ausgangsverbindung der Formel II als Piperidinium-Salz eingesetzt wird, kommen als Säureanionen in Frage: $Cl^-$, $Br^-$,$J^-$, Tosylat-Anion, Schwefelsäureanion ($HSO_4^-$, $SO_2^{2-}$)

Zu dem Verfahren c):
Diese Reaktion erfolgt in bekannter Weise ohne Lösungsmittel oder in einem inerten Lösungsmittel bei Temperaturen zwischen 50 bis 100°C.
Als Lösungsmittel kommen die gleichen in Frage wie für das Verfahren b); ebenfalls kann das Piperidin beziehungsweise N-Methyl-piperidin im Überschuß als Lösungsmittel wirken.

Zweckmäßig erfolgt die Umsetzung in Gegenwart von säurebindenden (halogenid-bindenden) Stoffen wie zum Beispiel $Ag_2CO_3$.

Zu dem Verfahren d):
Die Alkylierung erfolgt beispielsweise durch Umsetzung mit Verbindungen der Formel MHal, $ArSO_2OM$ und $SO_2(OM)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl- oder Naphthylrest und M der Methylrest ist. Beispiele sind p-Toluolsulfonsäure-methylester, Dimethylsulfat, Methylhalogenide. Die Alkylierungsreaktion wird gegebenenfalls unter Zusatz von üblichen säurebindenden Mitteln, wie Alkalihydroxiden, Alkalicarbonaten, Alkalihydrogencarbonaten, Erdalkalicarbonaten, Alkaliacetaten, tertiären Aminen (zum Beispiel Trialkylaminen wie Triethylamin), Pyridin oder auch Alkalihydriden bei Temperaturen zwischen 0 und 200°C, vorzugsweise 40 und 140°C in inerten Lösungsmitteln oder Suspensionsmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxide wie zum Beispiel Dimethylsulfoxid; tertiäre Säureamide wie zum Beispiel Dimethylformamid, N-Methyl-pyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungs- beziehungsweise Dispergiermittel.

Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100°C, vorzugsweise 20-80°C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Dimethylsulfoxid, Acetonitril, Dimethoxyethan, Aceton, Tetrahydrofuran.

Gegebenenfalls kann man bei der Alkylierung auch so vorgehen, daß man zuerst von der zu alkylierenden Verbindung eine Akaliverbindung (Natrium-, Kalium-oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150°C umsetzt und dann das alkylierende Agens zufügt.

Anstelle der angeführten Alkylierungsmittel können auch andere in der Chemie gebräuchliche chemisch äquivalente Mittel verwendet werden (siehe zum Beispiel auch L.F. und Mary Fieser "Reagents for Organic Synthesis", John Wiley and Sons, Inc., New York, 1967, Vol. 1, Seiten 1303-4 und Vol. 2, Seite 471).

Tabelle 1

| Substanz | Wachstumshemmungsindex WHI in % in vivo /humane KB-Tumorzellinie transplantiert an Nacktmäusen | $EC_{90}$ in µg/ml humane KB-Tumorzellinie in vitro | Cape-Index |
|---|---|---|---|
| $C_{18}H_{37}$-A- 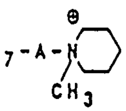 D 20133, erfindungsgemäße Verbindung | 121 | 0,3 | 403 |
| Verbindungen ähnlicher Struktur | | | |
| $C_{19}H_{39}$-A- 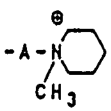 D21545 | 13,7 | 1,3 | 10 |
| $C_{20}H_{41}$-A- 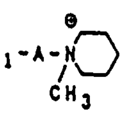 D 21033 | 7 | 0,3 | 23 |

$A = O{-}\underset{O^{\ominus}}{P}O{-}O{-}CH_2{-}CH_2{-}$

| | | | |
|---|---|---|---|
| $C_{22}H_{45}$-A- 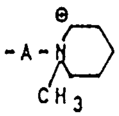 D 21052 | 0 | 3,2 | 0 |
| $C_{17}H_{35}$-A- 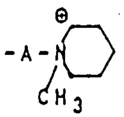 D 20813 | 76 | 2 | 38 |
| $C_{16}H_{33}$-A- 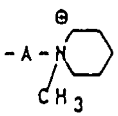 D 20317 | 38 | 1,4 | 27 |
| $C_{18}H_{37}$-A- 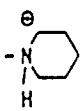 D 20110 | 25 | 1 | 25 |
| $C_{18}H_{37}$-A- 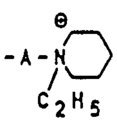 D 20931 | 13 | 1 | 13 |

| | | | | |
|---|---|---|---|---|
| $C_{18}H_{37}$-A- 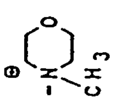 | D 20680 | 0 | >10 | 0 |
| $C_{18}H_{37}$-A- 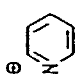 | D 19862 | 0 | > 1 | 0 |
| $C_{18}H_{37}$-A-$\overset{\oplus}{N}(CH_3)_3$ | D 19391 | 11 | 0,31 | 36 |
| $C_{18}H_{37}$-A- 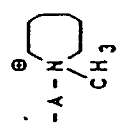 | D 20918 | 0 | 1 | 0 |
| $C_{18}H_{37}$-A- 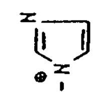 | D 20706 | 0 | 3,1 | 0 |
| $C_{18}H_{37}$-A- 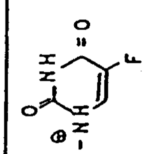 | D 20745 | 20 | 10 | 2 |

Der Wachstumshemmungsindex WHI wird bestimmt an der humanen KB-Tumorzellinie, die in Nacktmäuse transplantiert ist. Der Wachstumshemmungsindex ist wie folgt definiert:

$$WHI = \frac{M_{Kontrolle} - M_{Behandlung}}{M_{Kontrolle}} \times 100\ \%$$

der behandelten Gruppe beziehungsweise in der Kontrollgruppe am 14. Tag nach der Behandlung bezogen auf den Wert am Appllkationstag. Ein WHI größer als 100 % bedeutet, daß sich die Tumore wegen der Behandlung verkleinert haben; ein WHI kleiner als 100 % zeigt dagegen nur eine behandlungsbedingte Verlangsamung des Tumorwachstums gegenüber den Kontrollen an. Je größer der WHI, desto stärker also die antitumorale Wirksamkeit einer Testsubstanz.

Die $EC_{90}$ wird an derselben Zellinie in vitro bestimmt. Die $EC_{90}$ ist die Konzentration einer antitumorwirksamen Substanz in µg/ml, die in vitro das Wachstum der Krebszellen um 90 % hemmt im Vergleich zu einem Kontrollversuch ohne Zugabe der antitumorwirksamen Substanz.

Der Cape-Index (Koeffizient für krebshemmende Effekte) ist ein Maß für die hohe Selektivität und Spezifität einer chemischen Substanz gegenüber antitumorwirksamen Substanzen. Dieser Cape-Index wird dadurch erhalten, daß man den WHI-Index durch den $EC_{90}$-Index dividiert.

Das heißt eine Wirkung am Tier (in vivo) wird in Relation gesetzt zur Wirkung in einem in vitro-System. Es hat sich gezeigt, daß je höher der so gebildete Koeffizient ist, desto selektiver und wirksamer eine Substanz in ihrer Antitumorwirkung ist.

Die erfindungsgemäßen Verbindungen zeigen am KB-Tumor (humanes Carcinom der Mundhöhle, transplantiert subcutan auf Nacktmäuse) und am autochthonen, chemisch-induzierten DMBA-Brustdrüsenkrebs der Ratte eine gute antineoplastische Wirkung.

Beispielsweise wurde mit einer Dosis von 2 x 316 mg/kg, Körpergewicht der erfindungsgemäßen Verbindung, gegeben im Abstand von einer Woche, eine Regression der KB-Tumore erreicht.

Nach einer 14-tägigen oralen Behandlung mit einer oralen Dosis von 21,5 mg/kg Körpergewicht der erfindungsgemäßen Verbindung täglich regredierten 5 g schwere DMBA-Tumore bis an die Grenze der Tastbarkeit.

Wie die Tabelle 1 zeigt, besitzt also die erfindungsgemäße Verbindung im Vergleich zu den Verbindungen ähnlicher Struktur eine um ein Vielfaches höhere und spezifischere Antitumorwirksamkeit. Dieser Effekt war nicht naheliegend.

Beispiel 1

Herstellung nach dem neuen Verfahren (Verfahren a)

Octadecyl-[2-(N-methylpiperidino))-ethyl]-phosphat (Chiffre D-20133)

In einer Rührapparatur werden unter Stickstoff 9,2 ml (0,1 mol) $POCl_3$ in 50 ml Chloroform vorgelegt und im Eisbad auf 5 °C abgekühlt. 24.3 g (0,09 mol) Octadecanol werden in 100 ml Chloroform unter leichtem Erwärmen gelöst und zusammen mit 32 ml (0,40 mol) Pyridin bei einer Temperatur von 5 - 12 °C zugetropft. Zutropfzeit: 30 Minuten. Nach einer halben Stunde Nachrühren werden 37,9 (0,12 mol) festes 2-(N-Methyl-piperidino)-ethanol-Tosylat zugegeben und anschließend 40 ml Pyridin zugetropft. Die Temperatur steigt dabei auf 15 °C an. Das Eisbad wird entfernt und die Reaktionsmischung 2,5 Stunden bei Raumtemperatur gerührt. Zur Hydrolyse werden 15 ml Wasser innerhalb von 10 Minuten zugetropft. Nach einer halben Stunde Rühren wird die Reaktionslösung je einmal mit 200 ml Wasser/Methanol (1:1), 200 ml 3 proz. $Na_2CO_3$/Methanol (1:1) und 200 ml Wasser/Methanol (1:1) gewaschen. Die so gewaschene Reaktionslösung wird über $Na_2SO_4$ getrocknet und nach Zugabe von etwas i-Propanol im Vakuum einrotiert.

Der Rückstand wird aus 200 ml Butanon umkristallisiert. Das wachsartig ausfallende Produkt wird abfiltriert und in 150 ml 96%igem Ethanol heiß gelöst. Der Niederschlag wird abfiltriert und das Filtrat im Kühlschrank vier Stunden abgekühlt.

Der Niederschlag wird erneut abfiltriert und das Filtrat mit 85 g Amberlite® MB 3 versetzt. Nach drei Stunden Rühren wird der Ionenaustauscher entfernt und im Vakuum einrotiert. Der Rückstand wird aus 150 ml Butanon umkristallisiert. Trocknung im Vakuum über Phophorpentoxid.

Ausbeute: 21.7 g (45,6 mmol, 51 %).

F. 113°C

Rf-Wert: 0,53 (Dünnschichtchromatogramm an Kieselgel, Laufmittel Methylenchlorid/Methanol/25 prozentiges Ammoniak 80:25:5.

Die Herstellung der Ausgangs-N-Methyl -piperidino-Verbindung kann beispielsweise auf folgende Weise erfolgen: 64.59g (0.50mol) 2-Piperidinioethanol werden in 125mL Acetonitril gelöst und unter Kühlung tropfenweise mit einer Lösung von 93.1g (0.50mol) 4-Toluolsulfonsäuremethylester in 125ml Acetonitril innerhalb von 30 Minuten versetzt. Man rührt je eine halbe Stunde bei Raumtemperatur und unter Rückfluß nach. Nach dem Abkühlen zieht man das Lösungsmittel im Vakuum ab und löst das zurückbleibende Öl in 200mL heißem Aceton. Beim Abkühlen fällt das Produkt kristallin aus. Die Kristallisation wird im Eisfach vervollständigt. Nach dem Absaugen wird mit Aceton nachgewaschen und bei 40°C über $P_2O_5$ im Vakuum getrocknet.

Ausbeute: 139g (0.45 mol, 90%) 2-Hydroxyethyl-N-methylpiperidiniumtosylat.

Beispiel 2

Herstellung nach Verfahren c)

Octadecyl-[2-(N-methylpiperidino)-ethyl]-phosphat (Chiffre D-20133)

20.0g (44.0 mmol) Octadecyl-2-bromethylphosphat und 73ml (600 mmol) N-Methylpiperidin werden 3 Stunden unter Rückfluß erhitzt. Nach dem Abkühlen wird eingedampft, der Rückstand in 120ml Methanol aufgenommen und nach Zugabe von 17.9g (62.0 mmol) $Ag_2CO_3$ drei Stunden unter Rückfluß gekocht. Die Reaktionsmischung wird heiß über einen Membranfilter filtriert und eingeengt. Der Rückstand wird in 25 ml $CHCl_3$/Methanol/25 proz. $NH_4OH$ (80:25:5) gelöst und über eine Schicht Kieselgel filtriert. Das Filtrat wird eindampft, der Rückstand mit Aceton ausgerührt und abgesaugt. Das Produkt wird in wenig $CH_2Cl_2$ gelöst und mit Aceton ausgefällt.
Ausbeute: 3.50g (7.38mmol, 17%) weißes Pulver.
F.: 110-112°C
Rf-Wert: 0,53, Dünnschichtchromatogramm an Kieselgel, Laufmittel/Methanol/25 % Ammoniak 80:25:5.

Herstellung der Ausgangsverbindung:
Octadecyl-2-bromethyl-phosphat:
62.5g (0.50 mol) 2-Bromethanol werden in 50ml $CHCl_3$ gelöst und bei Raumtemperatur mit 68.7ml (0.75 mol) $POCl_3$ tropfenweise versetzt. Unter Argon wird 6 Stunden auf 55°C erhitzt und anschließend über Nacht stehen gelassen. Danach wird in Vakuum eingedampft und das gebildete 2-Bromethylphosphoroxydichlorid im Hochvakuum destilliert.
Ausbeute: 68.0g (281 mmol; 57%) 2-Bromethylphosphoroxydichlorid, Siedepunkt 58-62°C (0.1 mbar).

44.4 g (0.164 mol) Octadecanol und 60 g (0.249 mol) 2-Bromethylphosphoroxydichlorid werden in 250ml Toluol gelöst und unter Rühren tropfenweise mit 19.8ml (0.249 mol) Pyridin versetzt. Nach 4 Stunden Rühren wird eingedampft, der Rückstand mit 160ml $H_2O$ versetzt und 1.5 Stunden unter Rückfluß erhitzt. Die noch heiße Suspension wird auf 160g Eis, dem 41.5ml konzentrierte HCl zugesetzt wurden, gegossen. Das ausgefallene Octadecyl-2-bromethylphosphat wird abgesaugt und über $P_2O_5$ getrocknet.
Ausbeute: 64.5g (0.140 mmol, 86%) Octadecyl-2-bromethylphosphat.

Beispiel 3

Herstellung von Octadecyl-[2-(N-methylpiperidino-ethyl]-phosphat nach Verfahren b)

Ethylenoctadecylphosphat wird in 600ml Acetonitril gelöst und im Autoklaven 24 Stunden mit 125ml (1.03 mol) N-Methylpiperidin bei 80°C zur Reaktion gebracht. Nach dem Abkühlen wird die Apparatur geöffnet und die braune Reaktionslösung ins Eisfach zur Kristallisation gestellt. Nach dem Absaugen und Trocknen werden 92g (193 mmol, 61%) Reaktionsprodukt erhalten. Die Kristalle werden mit Aceton ausgerührt, in $CH_2Cl_2$ gelöst und an Kieselgel mit $CHCl_3$/Methanol/25 prozentiges $NH_4OH$ (60:40:4, danach 80:25:5) chromatographiert. Die das Produkt enthaltenden Fraktionen werden eingeengt, wobei das Octadecyl-[2-N-methylpiperidino)-ethyl]-phosphat nach dem Trocknen über $P_2O_5$ im Vakuum als weißer Feststoff anfällt.
F.: 108 - 110°C
Rf: 0,53, Dünnschichtchromatogramm an Kieselgel;
Laufmittel Methylenchlorid/Methanol/25%iges wässeriges Ammoniak 80:25:5
Ausbeute: 18g (37.8 mmol, 12%)
Das Ausgangs-Ethylenoctadecylphosphat wird zum Beispiel wie folgt erhalten:
85.3g (315 mmol) Octadecanol werden unter $N_2$ in einer trockenen Rührapparatur in 500ml trockenem Ether gelöst und mit 41ml (345 mmol) frisch destilliertem N-Methylpiperidin versetzt. Nach dem Abkühlen auf 5°C tropft man unter Feuchtigkeitsausschluß innerhalb von 30 Minuten eine Lösung aus 50g (345 mmol) 2-Chlor-2-oxo-1,3,2-dioxaphospholan in 170ml trockenem Ether bei 5-10°C zu. Nach beendeter Zugabe rührt man eine Stunde bei Raumtemperatur nach. Unter Schutzgas wird das ausgefallene N-Methylpiperidiniumhydrochlorid abgesaugt und das Filtrat bei 30°C Badtemperatur im Vakuum eingeengt. Ausbeute: 103g (284 mmol, 90%) Ethylenoctadecylphosphat.

Beispiel 4

Herstellung von Octadecyl-[2-(N-methylpiperidino-ethyl]-phosphat nach Verfahren d), Methylierung.

3.7g (8.00 mmol) Octadecyl-2-piperidinoethylphosphat und 0.63 ml (10.0 mmol) Methyljodid werden in 20ml Acetonitril gelöst und im Autoklaven 6 Stunden auf 100°C erhitzt. Man kühlt auf Raumtemperatur ab, saugt den entstandenen Niederschlag ab, löst in 140ml 96 prozentigem Ethanol und rührt 1.5 Stunden mit 50g Ionenaustauscher Amberlit®MB-3. Nach dem Abfiltrieren vom Ionenaustauscher wird eingedampft. Der Rückstand wird mit 20ml Butanon ausgerührt

und bei 40°C im Vakuum über $P_2O_5$ getrocknet.
Ausbeute: 0.96 g (2.02 mmol) amorphes Produkt D-20133

Die Ausganssubstanz wird beispielsweise wie folgt erhalten:
27.3g (60.0 mmol) Octadecyl-2-bromethylphosphat werden in 90ml (900 mmol) Piperidin gelöst und 1.5 Stunden unter Rückfluß gekocht. Nach dem Abkühlen dampft man ein, löst den Rückstand in 150ml Methanol, versetzt mit 25.2g (90.0 mmol) $Ag_2CO_3$ und kocht 1.5 Stunden. Es wird heiß über einen Membranfilter abgesaugt und eingeengt. Das erhaltene schwarze Öl wird an Kieselgel mit $CHCl_3$/Methanol/25 prozentigem $NH_4OH$ (80:25:5) chromatographiert. Die das Octadecyl-2-piperidinoethylphosphat enthaltenden Fraktionen werden eingeengt und der Rückstand aus 200ml Butanon umkristallisiert.
Ausbeute: 13.2g (28.6 mmol, 48%) Octadecyl-2-piperidinoethylphosphat.

**Patentansprüche**

**Patentansprüche für folgende Vertragsstaaten : AT, BE, CH, DE, DK, FR, GB, IT, LI, LU, MC, NL, PT, SE**

1. Arzneimittel mit antineoplastischer Wirkung enthaltend als Wirkstoff Octadecyl-[2-(N-methylpiperidino)-ethyl]-phosphat.

2. Verfahren zur Herstellung von Octadecyl-[2-(N-methylpiperidino)-ethyl]-phosphat, dadurch gekennzeichnet, daß man

   a) in einem Eintopfverfahren n-Octadecylalkohol mit Phosphoroxychlorid in einem inerten Lösungsmittel oder auch ohne Lösungsmittel in Gegenwart oder Abwesenheit einer basischen Substanz umsetzt, das erhaltene Produkt ohne Isolierung und Reinigung in einem inerten Lösungsmittel mit einem N-Methyl-piperidino-ethanol-Salz in Gegenwart einer basischen Substanz zum Phosphorsäurediesterchlorid weiter umsetzt und durch anschließende Hydrolyse das Octadecyl-[2-(N-methylpiperidino)-ethyl] -phosphat freisetzt und isoliert, oder daß man in an sich bekannter Weise

   b) eine Verbindung der Formel

$$C_{18}H_{37}\text{-O-PO(OH)}_2 \qquad \text{I}$$

   oder ein reaktionsfähiges Derivat dieser Verbindung mit einer Verbindung der Formel

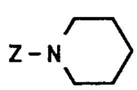

II

   gegebenenfalls in Gegenwart basischer Substanzen umsetzt, worin Z Wasserstoff, Methyl oder die Gruppe -$CH_2$-$CH_2$-OH darstellt und die Piperidinverbindung auch in Form des N-Methyl-piperidinium-Derivats vorliegen kann, wobei in diesem Falle die positive Ladung durch das Anion einer anorganischen oder organischen Säure neutralisiert wird und gegebenenfalls methyliert, oder

   c) eine Verbindung der Formel

$$C_{18}H_{37}\text{-O-PO(OH)-O-}CH_2\text{-}CH_2\text{-Y} \qquad \text{III}$$

   worin Y Chlor, Brom oder Jod ist mit Piperidin oder N-Methyl-piperidin umsetzt und gegebenenfalls methyliert oder

   d) eine Verbindung der allgemeinen Formel

$$C_{18}H_{37}\text{-O-PO(OH)-O-}CH_2\text{-}CH_2\text{-N} \qquad \text{IV}$$

   methyliert, beziehungsweise eine gemäß b) oder c) erhaltene Verbindung methyliert.

3. Reinigungsverfahren für das Octadecyl-[2-(N-methylpiperidino)-ethyl]-phosphat dadurch gekennzeichnet, daß man eine Lösung von Octadecyl-[2-(N-methylpiperidino)-ethyl]-phosphat, das mittels an und für sich bekannter Verfahren

oder nach dem Verfahren gemäß Anspruch 2 hergestellt wurde, in einem organischen Mittel mit einem Mischbettionenaustauscher, oder nacheinander mit einem sauren Ionenaustauscher und einem basischen Ionenaustauscher behandelt.

4. Octadecyl-[2-(N-methylpiperidino)-ethyl]-phosphat zur Anwendung als therapeutischer Wirkstoff.

5. Arzneimittel, enthaltend Octadecyl-[2-(N-methylpiperidino)-ethyl]-phosphat neben üblichen Träger- und/oder Verdünnungsbeziehungsweise Hilfsstoffen.

6. Verfahren zur Herstellung eines Arzneimittels, dadurch gekennzeichnet, daß Octadecyl-[2-(N-methylpiperidino)-ethyl]-phosphat mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

7. Verwendung von Octadecyl-[2-(N-methylpiperidino-ethyl]-phosphat zur Herstellung von Arzneimitteln.

**Patentansprüche für folgende Vertragsstaaten : ES, GR**

1. Verfahren zur Herstellung von Octadecyl-[2-(N-methylpiperidino)-ethyl]-phosphat, dadurch gekennzeichnet, daß man

a) in einem Eintopfverfahren n-Octadecylalkohol mit Phosphoroxychlorid in einem inerten Lösungsmittel oder auch ohne Lösungsmittel in Gegenwart oder Abwesenheit einer basischen Substanz umsetzt, das erhaltene Produkt ohne Isolierung und Reinigung in einem inerten Lösungsmittel mit einem N-Methyl-piperidino-ethanol-Salz in Gegenwart einer basischen Substanz zum Phosphorsäurediesterchlorid weiter umsetzt und durch anschließende Hydrolyse das Octadecyl-[2-(N-methylpiperidino)-ethyl] -phosphat freisetzt und isoliert, oder daß man in an sich bekannter Weise

b) eine Verbindung der Formel

$$C_{18}H_{37}\text{-O-PO(OH)}_2 \qquad \text{I}$$

oder ein reaktionsfähiges Derivat dieser Verbindung mit einer Verbindung der Formel

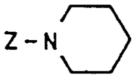

II

gegebenenfalls in Gegenwart basischer Substanzen umsetzt, worin Z Wasserstoff, Methyl oder die Gruppe -$CH_2$-$CH_2$-OH darstellt und die Piperidinverbindung auch in Form des N-Methyl-piperidinium-Derivats vorliegen kann, wobei in diesem Falle die positive Ladung durch das Anion einer anorganischen oder organischen Säure neutralisiert wird und gegebenenfalls methyliert, oder

c) eine Verbindung der Formel

$$C_{18}H_{37}\text{-O-PO(OH)-O-}CH_2\text{-}CH_2\text{-Y} \qquad \text{III}$$

worin Y Chlor, Brom oder Jod ist mit Piperidin oder N-Methyl-piperidin umsetzt und gegebenenfalls methyliert oder

d) eine Verbindung der allgemeinen Formel

$$C_{18}H_{37}\text{-O-PO(OH)-O-}CH_2\text{-}CH_2\text{-N} \qquad \text{IV}$$

methyliert, beziehungsweise eine gemäß b) oder c) erhaltene Verbindung methyliert.

2. Reinigungsverfahren für das Octadecyl-[2-(N-methylpiperidino)-ethyl]-phosphat dadurch gekennzeichnet, daß man eine Lösung von Octadecyl-[2-(N-methylpiperidino)-ethyl]-phosphat, das mittels an und für sich bekannter Verfahren oder nach dem Verfahren gemäß Anspruch 2 hergestellt wurde, in einem organischen Mittel mit einem Mischbettionenaustauscher, oder nacheinander mit einem sauren Ionenaustauscher und einem basischen Ionenaustauscher behandelt.

3. Verfahren zur Herstellung eines Arzneimittels, dadurch gekennzeichnet, daß Octadecyl-[2-(N-methylpiperidino)-ethyl]-phosphat mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

4. Verwendung von Octadecyl-[2-(N-methylpiperidinoethyl]-phosphat zur Herstellung von Arzneimitteln.

**Claims**

**Claims for the following Contracting States : AT, BE, CH, DE, DK, FR, GB, IT, LI, LU, MC, NL, PT, SE**

1. Medicine having antineoplastic action and containing as the active ingredient octadecyl-[2-(N-methylpiperidino)ethyl] phosphate.

2. Method for the preparation of octadecyl-[2-(N-methylpiperidino)ethyl] phosphate, characterised in that

a) in a one-pot process n-octadecyl alcohol is reacted with phosphorus oxychloride in an inert solvent or also without solvent in the presence or absence of a basic substance, the product obtained without being isolated or purified is further reacted in an inert solvent with an N-methylpiperidinoethanol salt in the presence of a basic substance to form the phosphate diester chloride, and octadecyl-[2-(N-methylpiperidino)ethyl] phosphate is liberated by subsequent hydrolysis and is isolated, or that in a known manner

b) a compound of the formula

$$C_{18}H_{37}\text{-O-PO(OH)}_2 \qquad (I)$$

or a reactive derivative of this compound is reacted with a compound of the formula

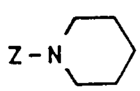

(II)

optionally in the presence of basic substances, wherein Z represents hydrogen, methyl or the group $-CH_2-CH_2-OH$ and the piperidine compound may also be present in the form of the N-methylpiperidinium derivative, with the positive charge in this case being neutralised by the anion of an inorganic or organic acid and optionally methylated, or

c) a compound of the formula

$$C_{18}H_{37}\text{-O-PO(OH)-O-}CH_2\text{-}CH_2\text{-Y} \qquad III$$

wherein Y represents chlorine, bromine or iodine, is reacted with piperidine or N-methylpiperidine and optionally methylated or

d) a compound of the general formula

$$C_{18}H_{37}\text{-O-PO(OH)-O-}CH_2\text{-}CH_2\text{-N} \qquad IV$$

is methylated, or a compound obtained as in b) or c) is methylated.

**3.** Purification method for the octadecyl-[2-(N-methylpiperidino)ethyl] phosphate, characterised in that a solution of octadecyl-[2-(N-methylpiperidino)ethyl] phosphate, which has been prepared by means of methods known per se or by the method according to claim 2, is treated in an organic medium using a mixed-bed ion exchanger, or successively using an acidic ion exchanger and a basic ion exchanger.

**4.** Octadecyl-[2-(N-methylpiperidino)ethyl] phosphate for application as a therapeutic agent.

**5.** Medicine, containing octadecyl-[2-(N-methylpiperidino)ethyl] phosphate in addition to conventional carriers and/or diluents or auxiliary substances.

**6.** Method for the preparation of a medicine, characterised in that octadecyl-[2-(N-methylpiperidino)ethyl] phosphate together with conventional used pharmaceutical carriers and/or diluents or other auxiliary substances is processed into pharmaceutical formulations or brought to a therapeutically usable form.

**7.** Use of octadecyl-[2-(N-methyl-piperidino)ethyl] phosphate for the preparation of medicines.

**Claims for the following Contracting States : ES, GR**

**1.** Method for the preparation of octadecyl-[2-(N-methylpiperidino)ethyl] phosphate, characterised in that

a) in a one-pot process n-octadeyl alcohol is reacted with phosphorus oxychloride in an inert solvent or also without solvent in the presence or absence of a basic substance, the product obtained without being isolated or purified is further reacted in an inert solvent with an N-methylpiperidinoethanol salt in the presence of a basic substance to form the phosphate diester chloride, and octadecyl-[2-(N-methylpiperidino)-ethyl] phosphate is liberated by subsequent hydrolysis and is isolated, or that in a known manner

b) a compound of the formula

$$C_{18}H_{37}\text{-O-PO(OH)}_2 \qquad \text{(I)}$$

or a reactive derivative of this compound is reacted with a compound of the formula

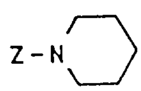

(II)

optionally in the presence of basic substances, wherein Z represents hydrogen, methyl or the group $-CH_2-CH_2-OH$ and the piperidine compound may also be present in the form of the N-methyl-piperidinium derivative, with the positive charge in this case being neutralised by the anion of an inorganic or organic acid and optionally methylated, or

c) a compound of the formula

$$C_{18}H_{37}\text{-O-PO(OH)-O-}CH_2\text{-}CH_2\text{-Y} \qquad \text{III}$$

wherein Y represents chlorine, bromine or iodine, is reacted with piperidine or N-methylpiperidine and optionally methylated or

d) a compound of the general formula

$$C_{18}H_{37}\text{-O-PO(OH)-O-}CH_2\text{-}CH_2\text{-N} \qquad \text{IV}$$

is methylated, or a compound obtained as in b) or c) is methylated.

**2.** Purification method for the octadecyl -[2-(N-methylpiperidino)-ethyl] phosphate, characterised in that a solution of octadecyl -[2-(N-methylpiperidino)-ethyl] phosphate, which has been prepared by means of methods known per se

or by the method according to claim 2, is treated in an organic medium using a mixed-bed ion exchanger, or successively using an acidic ion exchanger and a basic ion exchanger.

3. Method for the preparation of a medicine, characterised in that octadecyl-[2-(N-methylpiperidino)-ethyl] phosphate together with conventional used pharmaceutical carriers and/or diluents or other auxiliary substances is processed into pharmaceutical formulations or brought to a therapeutically usable form.

4. Use of octadecyl-[2-(N-methyl-piperidino)ethyl] phosphate for the preparation of medicines.

**Revendications**

**Revendications pour les Etats contractants suivants : AT, BE, CH, DE, DK, FR, GB, IT, LI, LU, MC, NL, PT, SE**

1. Médicament ayant une action antinéoplasique contenant comme principe actif l'octadécyl-[2-(N-méthylpipéridino)-éthyl]-phosphate.

2. Procédé d'obtention de l'octadécyl-[2-(N-méthylpipéridino)-éthyl]-phosphate, caractérisé en ce que l'on :

   a) fait réagir dans un procédé à un seul récipient, de l'alcool n-octadécylique avec l'oxychlorure de phosphore dans un solvant inerte ou aussi sans solvant, en présence ou en l'absence d'une substance basique, on fait réagir une seconde fois le produit obtenu sans isolement et sans purification dans un solvant inerte avec un sel de N-méthyl-pipéridino-éthanol en présence d'une substance basique en un chlorure de diester d'acide phosphorique et on libère par hydrolyse subséquente l'octadécyl-[2-(N-méthylpipéridino)-éthyl]-phosphate et on l'isole, ou
   b) fait réagir un composé de formule :

$$C_{18}H_{37}\text{-O-PO(OH)}_2 \quad (I)$$

   ou un dérivé apte à réagir de ce composé avec un composé de formule :

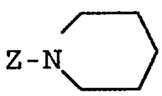

(II)

   le cas échéant en présence de substances basiques, dans laquelle Z représente de l'hydrogène, un méthyle ou le groupe $CH_2CH_2OH$ et la pipéridine peut se présenter aussi sous la forme du dérivé N-méthylpipéridinium, dans lequel dans ce cas la charge positive est neutralisée par l'anion d'un acide minéral ou organique et le cas échéant est méthylé, ou
   c) un composé de formule :

$$C_{18}H_{37}\text{-O-PO(OH)-O-}CH_2\text{-}CH_2\text{-Y} \quad (III)$$

   dans laquelle Y est un chlore, un brome ou un iode est mis à réagir avec la pipéridine ou la N-méthylpipéridine et le cas échéant méthylé, ou
   d) on méthyle un composé de formule générale :

$$C_{18}H_{37}\text{-O-PO(OH)-O-}CH_2CH_2\text{-N} \quad (IV)$$

   ou on méthyle un composé obtenu selon b) ou c).

3. Procédé de purification pour l'octadécyl-[2-(N-méthylpipéridino)-éthyl]-phosphate, caractérisé en ce que l'on traite une solution d'octadécyl-[2-(N-méthylpipéridino)-éthyl]-phosphate qui a été obtenu à l'aide d'un procédé connu en soi et pour soi, ou selon le procédé conformément à la revendication 2, par un moyen organique avec un échangeur d'ions en lit mélangé ou bien l'un après l'autre avec un échangeur d'ions acide puis un échangeur d'ions basique.

4. L'octadécyl-[2-(N-méthylpipéridino)-éthyl]-phosphate en vue de l'utilisation comme agent thérapeutique.

5. Médicament renfermant l'octadécyl-[2-(N-méthylpipéridino)-éthyl]-phosphate à côté de supports usuels et/ou d'agents de dilution ou d'autres substances auxiliaires.

6. Procédé d'obtention d'un médicament caractérisé en ce que l'octadécyl-[2-(N-méthylpipéridino)-éthyl]-phosphate avec des substances de support utilisés en pharmacie et/ou des agents de dilution ou des substances auxiliaires quelconques est utilisé après traitement dans des préparations pharmaceutiques ou sous une forme utilisable thérapeutiquement.

7. Utilisation de l'octadécyl-[2-(N-méthylpipéridino)-éthyl]-phosphate en vue de la production de médicaments.

**Revendications pour les Etats contractants suivants : ES, GR**

1. Procédé d'obtention de l'octadécyl-[2-(N-méthylpipéridino)-éthyl]-phosphate, caractérisé en ce que l'on:

   a) fait réagir dans un procédé à un seul récipient, de'alcool n-octadécylique avec l'oxychlorure de phosphore dans un solvant inerte ou aussi sans solvant, en présence ou en l'absence d'une substance basique, on fait réagir une seconde fois le produit obtenu sans isolement et sans purification dans un solvant inerte avec un sel de N-méthyl-pipéridino-éthanol en présence d'une substance basique en un chlorure de diester d'acide phosphorique et on libére par hydrolyse subséquente l'octadécyl-[2-(N-méthylpipéridino)-éthyl]-phosphate et on l'isole, ou

   b) fait réagir un composé de formule:

$$C_{18}H_{37}O\text{-}PO(OH)_2 \qquad (I)$$

   ou un dérivé apte à réagir de ce composé avec un composé de formule:

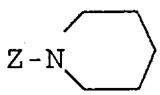

(II)

   le cas échéant en présence de substances basiques, dans laquelle Z représente de l'hydrogène, un méthyle ou le groupe $CH_2CH_2OH$ et la pipéridine peut se présenter aussi sous la forme du dérivé N-méthylpipéridinium, dans lequel dans ce cas la charge positive est neutralisée par l'anion d'un acide minéral ou organique et le cas échéant est méthylé, ou

   c) un composé de formule:

$$C_{18}H_{37}\text{-}O\text{-}PO(OH)\text{-}O\text{-}CH_2\text{-}CH_2\text{-}Y \qquad (III)$$

   dans laquelle Y est un chlore, un brome ou un iode est mis à réagir avec la pipéridine ou la N-méthylpipéridine et le cas échéant méthylé, ou

   d) on méthyle un composé de formule générale:

$$C_{18}H_{37}\text{-}O\text{-}PO(OH)\text{-}O\text{-}CH_2CH_2\text{-}N \qquad (IV)$$

   ou on méthyle un composé obtenu selon b) ou c).

2. Procédé de purification pour l'octadécyl-[2-(N-méthylpipéridino)-éthyl]-phosphate, caractérisé en ce que l'on traite une solution d'octadécyl-[2 (N-méthylpipéridino)-éthyl]-phosphate qui a été obtenu à l'aide d'un procédé connu en soi et pour soi, ou selon le procédé conformément à la revendication 2, par un moyen organique avec un échangeur dons en lit mélangé ou bien l'un aprés l'autre avec un échangeur d'ions acide puis un échangeur d'ions basique.

3. Procédé d'obtention d'un médicament caractérisé en ce que l'octadécyl-[2-(N-méthylpipéridino)-éthyl]-phosphate avec des substances de support utilisés en pharmacie et/ou des agents de dilution ou des substances auxiliaires quelconques est utilisé aprés traitement dans des préparations pharmaceutiques ou sous une forme utilisable thérapeutiquement.

4. Utilisation de l'octadécyl-[2-(N-méthylpipéridino)-éthyl]-phosphate en vue de la production de médicaments.